# EUROPEAN PATENT SPECIFICATION

(11) **EP 3 354 299 B1**
(45) Date of publication and mention of the grant of the patent: **16.09.2020**
(21) Application number: 16848390.7
(22) Date of filing: 20.07.2016
(51) Int. Cl.: A61M 1/18

(54) **ARTIFICIAL LUNG**
KÜNSTLICHE LUNGE
POUMON ARTIFICIEL

(30) Priority: 25.09.2015 JP 2015188693
(43) Date of publication of application: 01.08.2018
(73) Proprietor: Terumo Kabushiki Kaisha, Tokyo 151-0072 (JP)
(72) Inventor: HISAMATSU, Kozo, Kanagawa 259-0151 (JP); SASAKI, Eisuke, Elkton, Maryland 21921 (US)
(74) Representative: Casalonga
(86) International application number: PCT/JP2016/071323
(87) International publication number: WO 2017/051600

(56) References cited:
- WO-A1-91/04758
- WO-A1-91/04758
- WO-A1-2015/128886
- WO-A1-2015/128886
- WO-A1-2016/136711
- WO-A2-2009/098457

## Description

### Technical Field

The present invention relates to an artificial lung,

In particular, the present invention relates to an artificial lung according to the preamble of claim 1, such as it is for example known from WO 91/04758, WO 2009/098457 or WO 2015/128886 A1.

### Background Art

In the related art, in cardiotomy for heart disease performed through an extracorporeal circulation method, an artificial lung has been used for replacing the function of a lung of a living body. In this case, blood of a patient is introduced into an artificial lung outside the body. In order to reduce the blood transfusion amount of a patient and to reduce adverse reaction caused due to blood transfusion, the filling amount of blood in an artificial lung is required to be reduced.

Various attempts have been made in this regard. For example, according to the artificial lung disclosed in PTL 1, the filling amount of blood is reduced by reducing a storage space for blood.

### Citation List

### Patent Literature

PTL 1: JP-A-2010-200884

### Summary of Invention

### Technical Problem

However, in the related art, a heat exchange portion which controls the temperature of blood and a gas exchange portion which performs gas exchange are separately provided inside a housing to be filled with blood, and a gap therebetween becomes a dead space.

In some artificial lungs in the related art, a partition wall is provided in a gap between the heat exchange portion and the gas exchange portion. Although a dead space is slightly reduced due to the partition wall, since blood moves between the heat exchange portion and the gas exchange portion, a large window-shaped opening portion is formed in the partition wall. Therefore, a large dead space still remains in the present circumstances.

Moreover, in some artificial lungs in the related art, a header having a partition wall with a thickness greater than a gap between a heat exchange layer and a gas exchange layer constituted of hollow fibers is forcibly inserted and is disposed therebetween. In this case, the hollow fibers of the heat exchange layer and the gas exchange layer are squashed by the partition wall of the header. As a result, there is a possibility that heat exchange performance and gas exchange performance will deteriorate.

The present invention has been made in consideration of the foregoing problems, and an object thereof is to provide an artificial long in which a filling amount of blood is more effectively reduced and a function of the artificial lung can be favorably exhibited.

### Solution to Problem

In order to achieve the object, according to the present invention, there is provided an artificial lung

The above problem is solved by an artificial lung according to claim 1. The dependent claims relate to advantageous embodiments.

### Advantageous Effects of Invention

According to the artificial lung having the configuration described above, the gap between the heat exchange portion and the gas exchange portion is filled with the porous member, and a useless space inside the filling portion to be filled with blood is reduced. Therefore, the filling amount of blood can be effectively reduced. In addition, blood moves between the heat exchange portion and the gas exchange portion through holes of the porous member, and a heat exchange and a gas exchange are smoothly performed. Therefore, the function of the artificial lung can be favorably exhibited.

### Brief Description of Drawings

[Fig. 1] Fig. 1 is a perspective view of an artificial lung of an embodiment.
[Fig. 2] Fig. 2 is a sectional view taken along line 2-2 in Fig. 1.
[Fig. 3A] Fig. 3A is an enlarged view illustrating a part of the reference sign 3 in Fig. 2.
[Fig. 3B] Fig. 3B is a plan view taken along line B-B in Fig. 3A.
[Fig. 4] Fig. 4 is a graph illustrating a relationship between a mesh opening dimension of a porous member and passing pressure of air bubbles.

### Description of Embodiment

Hereinafter, with reference to the accompanying drawings, an embodiment of the present invention will be described. Note that, for the convenience of description, the dimensional ratios of the drawings are exaggerated and are different from the actual ratios.

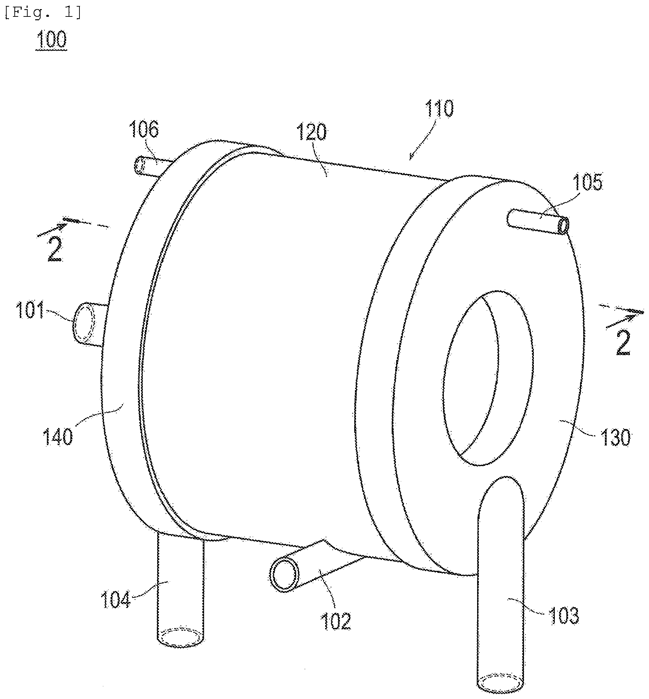

As illustrated in Fig. 1, an artificial lung 100 of the embodiment has a housing 110 (filling portion), an inlet port 101 and an outlet port 102 for blood, an inlet port 103 and an outlet port 104 for a heat transfer medium, and an inlet port 105 and an outlet port 106 for gas. The housing 110 has an outer cylindrical member 120, a header 130, and a header 140.

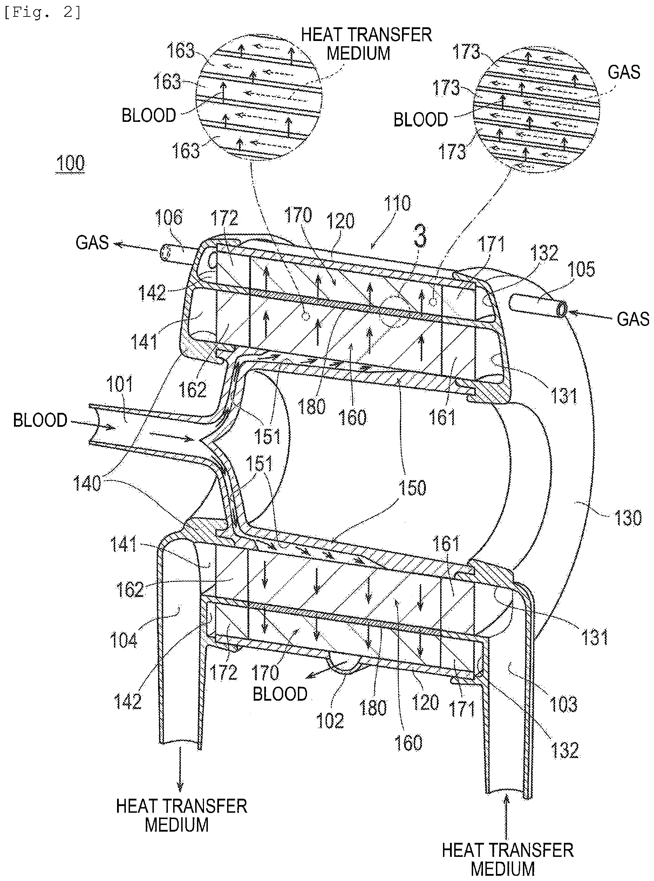

In addition, as illustrated in Fig. 2, the housing 110 has an inner cylindrical member 150. The outer cylindrical member 120 is provided to surround the inner cylindrical member 150.

A flow path 151 communicating with the inlet port 101 for blood is formed in the inner cylindrical member 150, and the outlet port 102 for blood is formed in the outer cylindrical member 120. The header 130 is attached to one end portion of the outer cylindrical member 120 and the inner cylindrical member 150, and the header 140 is attached to the other end portion of the outer cylindrical member 120 and the inner cylindrical member 150.

The inlet port 103 for a heat transfer medium and an inlet path 131 for a heat transfer medium are formed in the header 130. The inlet port 103 for a heat transfer medium and the inlet path 131 for a heat transfer medium communicate with each other.

In addition, the inlet port 105 for gas and an inlet path 132 for gas are formed in the header 130. The inlet port 105 for gas and the inlet path 132 for gas communicate with each other. The inlet path 131 for a heat transfer medium and the inlet path 132 for gas are isolated so as not to communicate with each other.

The outlet port 104 for a heat transfer medium and an outlet path 141 for a heat transfer medium are formed in the header 140. The outlet port 104 for a heat transfer medium and the outlet path 141 for a heat transfer medium communicate with each other.

In addition, the outlet port 106 for gas and an outlet path 142 for gas are formed in the header 140. The outlet port 106 for gas and the outlet path 142 for gas communicate with each other. The outlet path 141 for a heat transfer medium and the outlet path 142 for gas are isolated so as not to communicate with each other.

The housing 110 internally includes a heat exchange portion 160, a gas exchange portion 170, and a porous member 180.

The heat exchange portion 160 cylindrically extends around the inner cylindrical member 150. One end portion 161 of the heat exchange portion 160 is fixed to the inlet path 131 for a heat transfer medium using an adhesive, for example. The other end portion 162 of the heat exchange portion 160 is fixed to the outlet path 141 for a heat transfer medium using an adhesive, for example.

The gas exchange portion 170 is provided to be adjacent to the heat exchange portion 160 and cylindrically extends around the heat exchange portion 160. One end portion 171 of the gas exchange portion 170 is fixed to the inlet path 132 for gas using an adhesive, for example. The other end portion 172 of the gas exchange portion 170 is fixed to the outlet path 142 for gas using an adhesive, for example.

The porous member 180 is disposed between the heat exchange portion 160 and the gas exchange portion 170 and fills a gap therebetween. The porous member 180 fills the entire gap between the heat exchange portion 160 and the gas exchange portion 170. A material forming the porous member 180 is not particularly limited. For example, a resin having biocompatibility is used.

Blood introduced through the inlet port 101 for blood fills the inside of the housing 110. The heat exchange portion 160 performs temperature control, and the gas exchange portion 170 performs gas exchange.

Blood which has been introduced from the inlet port 101 for blood passes through the flow path 151 and is guided to the heat exchange portion 160. Blood moves radially outward through the heat exchange portion 160, the porous member 180, and the gas exchange portion 170.

The heat exchange portion 160 is constituted of a bundle of a plurality of hollow fibers 163 (hollow fibers for heat exchange), and blood passes through the heat exchange portion 160 through gaps among the hollow fibers 163.

Each of the hollow fibers 163 extends from a side of the inlet path 131 for a heat transfer medium to a side of the outlet path 141 for a heat transfer medium in a substantially straight manner. Each of the hollow fibers 163 communicates with the inlet path 131 for a heat transfer medium at one end portion and communicates with the outlet path 141 for a heat transfer medium at the other end portion.

A heat transfer medium is introduced from the inlet port 103 for a heat transfer medium and enters the inside of the hollow fibers 163 through the inlet path 131 for a heat transfer medium. The heat transfer medium which has flowed inside the hollow fibers 163 goes out to the outlet path 141 for a heat transfer medium and flows out from the outlet port 104 for a heat transfer medium.

Blood comes into contact with the hollow fibers 163 while moving through the gaps among the hollow fibers 163 and is subjected to heat exchange with the heat transfer medium flowing inside the hollow fibers 163. For example, the heat transfer medium is warm water or cold water controlled to have a predetermined temperature. However, the heat transfer medium is not limited thereto.

In the end portions 161 and 162 of the heat exchange portion 160, for example, the gaps among the hollow fibers 163 are in a liquid-tight state filled with an adhesive. Therefore, blood does not flow out to the inlet path 131 and the outlet path 141 for a heat transfer medium. In addition, the heat transfer medium does not enter the gaps among the hollow fibers 163 and is not mixed with blood.

The gas exchange portion 170 is constituted of a bundle of a plurality of hollow fibers 173 (hollow fibers for gas exchange), and blood passes through the gas exchange portion 170 through gaps among the hollow fibers 173. The diameter of a hollow fiber 173 is smaller than the diameter of a hollow fiber 163.

Each of the hollow fibers 173 extends from a side of the inlet path 132 for gas to a side of the outlet path 142 for gas in a substantially straight manner. Each of the hollow fibers 173 communicates with the inlet path 132 for gas at one end portion and communicates with the outlet path 142 for gas at the other end portion.

Gas is introduced from the inlet port 105 for gas and enters the inside of the hollow fibers 173 through the inlet path 132 for gas. The gas which has flowed inside the hollow fibers 173 goes out to the outlet path 142 for gas and flows out from the outlet port 106 for gas.

Blood comes into contact with the hollow fibers 173 while moving through the gaps among the hollow fibers 173. Micro-holes for internal communication are formed in a surrounding wall of the hollow fibers 173. When blood comes into contact with the hollow fibers 173, oxygen, that is, gas flowing inside the hollow fibers 173 is taken into blood through the holes. In addition, at this time, carbon dioxide in blood is taken into the hollow fibers 173.

In the end portions 171 and 172 of the gas exchange portion 170, for example, the gaps among the hollow fibers 173 are in a liquid-tight state filled with an adhesive. Therefore, blood does not flow out to the inlet path 132 and the outlet path 142 for gas. In addition, gas does not enter the gaps among the hollow fibers 173 and is not mixed with blood.

Blood is suitably subjected to temperature control and gas exchange through the heat exchange portion 160 and the gas exchange portion 170. Thereafter, the blood flows out through the outlet port 102 for blood.

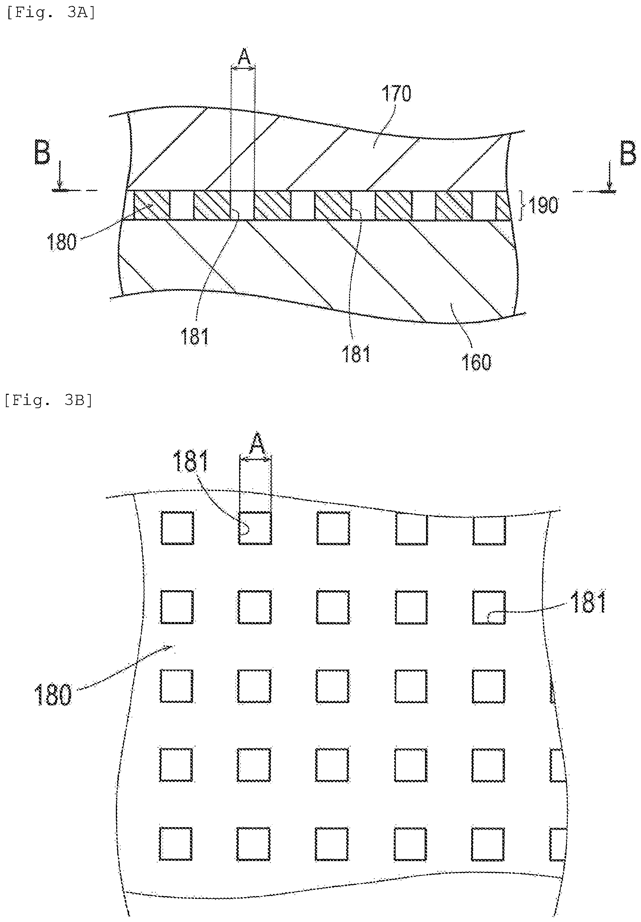

As illustrated in Fig. 3, the porous member 180 is a mesh material, in which the heat exchange portion 160 side and the gas exchange portion 170 side communicate with each other through holes 181, blood moves therebetween.

The porous member 180 reduces a useless space, suppresses the blood filling amount, and enables blood to move by filling a gap 190 between the heat exchange portion 160 and the gas exchange portion 170.

The opening ratio of the holes 181 indicates a ratio of the area of a mesh opening part per unit area.

If the mesh opening dimension A is reduced, the volume of the porous member 180 filling the gap 190 increases and an effect of reducing the blood filling amount becomes significant. However, resistance when blood passes through the holes 181 increases. As a factor increasing resistance at this time, air in blood is clogged in the holes 181 of which the mesh opening dimension A is reduced, and blood is inhibited from passing through. In addition, when resistance in the holes 181 increases and blood is hindered from smoothly flowing, blood becomes stagnant in the heat exchange portion 160 and the gas exchange portion 170, so that there is a possibility that heat exchange and gas exchange will not be favorably performed, and heat exchange performance and gas exchange performance will deteriorate.

Meanwhile, although resistance in the holes 181 is reduced by increasing the mesh opening dimension A, volume for filling the gap 190 is reduced. Therefore, an effect of reducing the blood filling amount deteriorates.

In this manner, the mesh opening dimension A and reduction of the blood filling amount are in a trade-off relationship, and the inventors calculated and verified the relationship. The calculation result is indicated in Table 1 below.

**[Table 1]**

| | Calculation Example 1 | Calculation Example 2 | Calculation Example 3 | Calculation Example 4 | Calculation Example 5 |
|---|---|---|---|---|---|
| Mesh opening dimension A (µm) | 33 | 100 | 200 | 840 | 1,800 |
| Opening ratio (%) | 21 | 32 | 43 | 46 | 61 |
| Reduction amount of filling blood (mL) | 11.4 | 9.8 | 8.2 | 7.8 | 4.3 |
| Heat exchange performance | Relatively low compared to Calculation Examples 3 to 5 | Relatively low compared to Calculation Examples 3 to 5 | Preferable | More preferable | More preferable |

The reduction amounts of filling blood indicated in Table 1 were obtained from the volume of the porous member 180, and it was considered that the amount of filling blood was reduced as much as the volume of the porous member 180. The volume of the porous member 180 was obtained by multiplying the superficial surface area of the porous member 180 including the mesh opening part of the holes 181 by the thickness of the porous member 180, and subtracting the total volume of the holes 181 therefrom. Here, the thickness of the porous member 180 was calculated as 1 mm. In addition, the total volume of the holes 181 was obtained by multiplying the volume of each of the holes 181 by the total number of the holes 181.

The heat exchange performance in Table 1 was evaluated based on a temperature change of blood at the inlet port 101 and the outlet port 102 for blood and a temperature change of a heat transfer medium at the inlet port 103 and the outlet port 104 for a heat transfer medium in the artificial lung 100.

The heat exchange performance of Calculation Examples 1 and 2 was within a permissible range but was inferior to those of Calculation Examples 3 to 5. Meanwhile, the heat exchange performance of Calculation Examples 3 to 5 was favorable.

It was assumed as a reason that when the mesh opening dimension A was significant such as 200 µm or greater as in Calculation Example 3 to 5, flow resistance in the holes 181 was reduced, blood flowed smoothly, and heat exchange was favorably performed in the heat exchange portion 160.

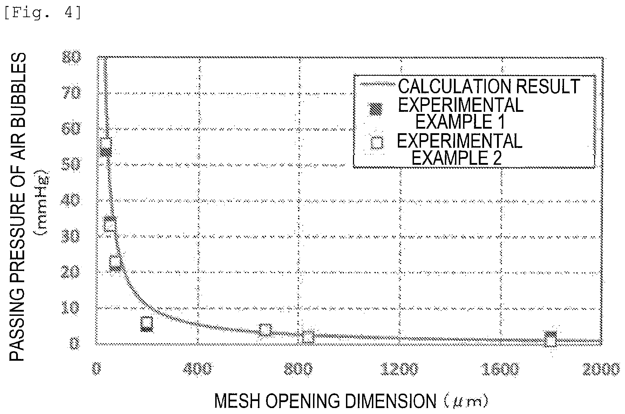

Actually, as illustrated in the graph of Fig. 4, even in the experimental result, when the mesh opening dimension A was 200 µm or greater, reduction of passing pressure of air bubbles was checked. From this reason as well, it is assumed that when the mesh opening dimension A is caused to be 200 µm or greater, blood flows smoothly without causing air bubbles to obstruct the flow path in the holes 181, and heat exchange and gas exchange are particularly and favorably performed. Here, passing pressure of air bubbles is pressure required for air bubbles to pass through the porous member 180. In the experiment, the pressure required for air bubbles to pass through the porous member 180 was measured while changing the mesh opening dimension A of the porous member 180. In addition, the quality of the material of the porous member 180 was changed in Experimental Example 1 and Experimental Example 2 in the graph of Fig. 4.

From the result of the calculation and the experiment, it is assumed that performance becomes preferable when the mesh opening dimension A ranges from 200 µm to 1,800 µm and the opening ratio ranges from 40% to 60%.

In addition, in regard to reduction of the blood filling amount as well, the filling amount of blood to fill the gas exchange portion 170 was approximately 60 mL. On the other hand, 4.3 mL was reduced in Calculation Example 5 having the least reduction amount of blood. Accordingly, it was found that at least 7% or higher reduction rate could be obtained.

The reduction rate of the blood filling amount is obtained based on the ratio of the filling amount of blood to fill the gas exchange portion 170 and the volume of a substrate part of the porous member 180 excluding the holes 181. The reduction rate of the blood filling amount is preferably 10% or higher. However, the reduction rate is not limited thereto. In addition, the upper limit for the reduction rate of the blood filling amount is not particularly limited. For example, the upper limit is 20% or lower.

Next, an operational effect of the present embodiment will be described.

According to the artificial lung 100 of the present embodiment, the gap 190 between the heat exchange portion 160 and the gas exchange portion 170 is filled with the porous member 180, and a useless space inside the housing 110 to be filled with blood is reduced. Therefore, the filling amount of blood can be effectively reduced. In addition, blood moves between the heat exchange portion 160 and the gas exchange portion 170 through the holes 181 of the porous member 180, and a heat exchange and a gas exchange are smoothly performed. Therefore, the function of the artificial lung 100 can be favorably exhibited.

When the mesh opening dimension A of the holes 181 ranges from 200 µm to 1,800 µm and the opening ratio ranges from 40% to 60%, resistance in the holes 181 is particularly and effectively suppressed, and a flow of blood is unlikely to be hindered. Therefore, it is possible to more reliably exhibit favorable heat exchange performance and gas exchange performance.

In addition, when the reduction rate of the blood filling amount due to the porous member 180 is 10% or higher, blood to fill the artificial lung 100 can be particularly and effectively reduced. Therefore, the blood transfusion amount with respect to a patient can be suppressed and a low-invasive technique can be performed.

The present invention is not limited to the embodiment described above and can be variously changed within the scope of Claims.

For example, in the embodiment, the housing 110, the heat exchange portion 160, the gas exchange portion 170, and the porous member 180 have a cylindrical shape. However, the shape thereof is not particularly limited. For example, the present invention includes a form in which a housing has a hollow rectangular parallelepiped shape, and a heat exchange portion having a flat rectangular shape, a porous member, and a gas exchange portion are stacked in this order inside thereof.

In addition, the porous member is not limited to a punching mesh obtained by forming a plurality of holes in a thin material. For example, the porous member may be a woven net formed with warp and weft. In addition, the porous member may be a porous body such as a sponge.

This application is based on Japanese Patent Application No. 2015-188693, filed on September 25, 2015.

### Reference Signs List

100 ARTIFICIAL LUNG,
101 INLET PORT FOR BLOOD,
102 OUTLET PORT FOR BLOOD,
103 INLET PORT FOR HEAT TRANSFER MEDIUM,
104 OUTLET PORT FOR HEAT TRANSFER MEDIUM,
105 INLET PORT FOR GAS,
106 OUTLET PORT FOR GAS,
110 HOUSING (FILLING PORTION),
120 OUTER CYLINDRICAL MEMBER,
130 HEADER,
131 INLET PATH FOR HEAT TRANSFER MEDIUM,
132 INLET PATH FOR GAS,
140 HEADER,
141 OUTLET PATH FOR HEAT TRANSFER MEDIUM,
142 OUTLET PATH FOR GAS,
150 INNER CYLINDRICAL MEMBER,
151 FLOW PATH FOR BLOOD FLOW,
160 HEAT EXCHANGE PORTION,
161, 162 END PORTION OF HEAT EXCHANGE PORTION,
163 HOLLOW FIBER FOR HEAT EXCHANGE,
170 GAS EXCHANGE PORTION,
171, 172 END PORTION OF GAS EXCHANGE PORTION,
173 HOLLOW FIBER FOR GAS EXCHANGE,
180 POROUS MEMBER,
181 HOLE OF POROUS MEMBER,
190 GAP BETWEEN HEAT EXCHANGE PORTION AND GAS EXCHANGE PORTION, AND
A MESH OPENING DIMENSION OF HOLE OF POROUS MEMBER.

## Claims

1. An artificial lung (100) comprising:
a filling portion (110) communicating with an inlet port (101) and an outlet port (102) for blood and configured to be filled with blood;
a heat exchange portion (160) including a bundle of a plurality of hollow fibers (163) for heat exchange and disposed in the filling portion (110);
a gas exchange portion (170) including a bundle of a plurality of hollow fibers (173) for gas exchange and disposed in the filling portion (110) to be adjacent to the heat exchange portion (160); and
a porous member (180) disposed in a gap (190) between the heat exchange portion (160) and the gas exchange portion (170) and filling the gap (190)
wherein the porous member (180) is a mesh material, in which a heat exchange portion side and a gas exchange portion side communicate with each other through holes (181) and blood moves therebetween,
**characterized in that** a mesh opening dimension (A) of a hole (181) formed in the porous member (180) ranges from 200 µm to 1,800 µm, and an opening ratio of the holes (181) ranges from 40% to 60%.

2. The artificial lung (100) according to Claim 1,
wherein a reduction rate of a blood filling amount due to the porous member (180) is 10% or higher.

## Patentansprüche

1. Künstliche Lunge (100), umfassend:
einen Füllabschnitt (110), der mit einer Einlassöffnung (101) und einer Auslassöffnung (102) für Blut in Verbindung steht und so konfiguriert ist, dass er mit Blut gefüllt werden kann;
einen Wärmeaustauschabschnitt (160), der ein Bündel aus einer Vielzahl von Hohlfasern (163) zum Wärmeaustausch umfasst und in dem Füllabschnitt (110) angeordnet ist;
einen Gasaustauschabschnitt (170), der ein Bündel aus einer Vielzahl von Hohlfasern (173) zum Gasaustausch umfasst und in dem Füllabschnitt (110) so angeordnet ist, dass er an den Wärmeaustauschabschnitt (160) angrenzend ist; und
ein poröses Element (180), das in einem Spalt (190) zwischen dem Wärmeaustauschabschnitt (160) und dem Gasaustauschabschnitt (170) angeordnet ist und den Spalt (190) ausfüllt,
wobei das poröse Element (180) ein Maschenmaterial ist, bei dem eine Wärmeaustauschabschnittsseite und eine Gasaustauschabschnittsseite durch Löcher (181) miteinander in Verbindung stehen und Blut sich dazwischen bewegt,
**dadurch gekennzeichnet, dass** eine Maschenöffnungsabmessung (A) eines in dem porösen Element (180) ausgebildeten Lochs (181) in dem Bereich von 200 µm bis 1.800 µm liegt und ein Öffnungsverhältnis der Löcher (181) in dem Bereich von 40 % bis 60 % liegt.

2. Künstliche Lunge (100) nach Anspruch 1,
wobei eine Reduktionsrate einer Blutfüllmenge aufgrund des porösen Elements (180) 10 % oder höher ist.

## Revendications

1. Poumon artificiel (100) comprenant :
une partie de remplissage (110) communiquant avec un orifice d'entrée (101) et un orifice de sortie (102) pour le sang et configurée pour être remplie de sang ;
une partie d'échange de chaleur (160) comportant un faisceau d'une pluralité de fibres creuses (163) pour un échange de chaleur et disposée dans la partie de remplissage (110) ;
une partie d'échange de gaz (170) comportant un faisceau d'une pluralité de fibres creuses (173) pour un échange de gaz et disposée dans la partie de remplissage (110) pour être adjacente à la partie d'échange de chaleur (160) ; et
un élément poreux (180) disposé dans un espace (190) entre la partie d'échange de chaleur (160) et la partie d'échange de gaz (170) et remplissant l'espace (190) dans lequel l'élément poreux (180) est un matériau à mailles, dans lequel un côté partie d'échange de chaleur et un côté partie d'échange de gaz communiquent l'un avec l'autre à travers des trous (181) et le sang se déplace entre ceux-ci,
**caractérisé en ce qu'**une dimension d'ouverture de maille (A) d'un trou (181) formé dans l'élément poreux (180) se trouve dans la plage de 200 µm à 1800 µm, et un taux d'ouverture des trous (181) se trouve dans la plage de 40 % à 60 %.

2. Poumon artificiel (100) selon la revendication 1,
dans lequel un taux de réduction d'une quantité de remplissage de sang dû à l'élément poreux (180) est de 10 % ou plus.
